Europäisches Patentamt

European Patent Office

Office européen des brevets

(11) Veröffentlichungsnummer: **0 094 049**
**B1**

(12) **EUROPÄISCHE PATENTSCHRIFT**

(45) Veröffentlichungstag der Patentschrift:
18.01.89

(51) Int. Cl.⁴: **A 61 K 6/08**

(21) Anmeldenummer: **83104449.0**

(22) Anmeldetag: **05.05.83**

(54) **Zahnfüllmasse, enthaltend einen Zusatz von Polyvinylbutyral.**

(30) Priorität: **07.05.82 DE 3217259**

(43) Veröffentlichungstag der Anmeldung:
**16.11.83 Patentblatt 83/46**

(45) Bekanntmachung des Hinweises auf die Patenterteilung:
**18.01.89 Patentblatt 89/3**

(84) Benannte Vertragsstaaten:
**AT BE CH DE FR GB IT LI NL SE**

(56) Entgegenhaltungen:
**CH-A- 610 199**
**DE-A- 1 006 125**
**DE-B- 1 202 937**
**GB-A- 2 041 954**
**US-A- 3 047 408**

**Adhesion and Adhesives, R. Houwink and G. Salomon, 2nd ed., Vol. 1, 1965, S. 300**

**Die Akte enthält technische Angaben, die nach dem Eingang der Anmeldung eingereicht wurden und die nicht in dieser Patentschrift enthalten sind.**

(73) Patentinhaber: **ESPE Stiftung & Co Produktions- und Vertriebs KG, D-8031 Seefeld (DE)**

(72) Erfinder: **Jochum, Peter, Dr., Pointweg 5, D-8031 Hechendorf (DE)**
Erfinder: **Gasser, Oswald, Dr., Hartstrasse 13, D-8031 Seefeld (DE)**

(74) Vertreter: **Strehl, Schübel-Hopf, Groening, Schulz et al, Maximilianstrasse 54 Postfach 22 14 55, D-8000 München 22 (DE)**

**Beschreibung**

Die Erfindung betrifft eine Zahnfüllmasse auf Basis von Calciumhydroxid und Salicylsäureestern mit einem Zusatz von Polyvinylbutyral, welche sich für Unterfüllungen und für die Pulpenüberkappung eignet.

Wegen der Toxizität der meisten üblichen Füllungen sind für tiefergehende Zahnkavitäten und insbesondere dann, wenn eine Pulpenüberkappung nötig wird, Unterfüllungen erforderlich.

Man hat für die Behandlung von Zahnkavitäten seit längerer Zeit Dispersionen von Calciumhydroxid in Wasser oder in wässrigen oder organischen Lösungen von filmbildenden Materialien eingesetzt, da Calciumhydroxid einerseits die Pulpa gegen das Eindringen von Säuren aus den Füllungen schützt, andererseits die Bildung von Sekundärdentin stimuliert.

Die Anwendung von Calciumhydroxid-Dispersionen in wässrigen oder organischen Systemen ergibt jedoch keine Unterfüllung mit ausreichender mechanischer Festigkeit, welche den Zahn vor den mechanischen Einwirkungen beim Einbringen der Füllung schützt.

Zur Beseitigung dieser Schwierigkeit wird in der US-A-3 047 408 eine Zahnfüllmasse beschrieben, die aus pulverförmigem Calciumhydroxid und Estern aus mehrwertigen Alkoholen mit Salicylsäure sowie gegebenenfalls Methylsalicylat, inerten gepulverten Füllstoffen und/oder Weichmachern erhalten wird.

Dieser Dentalzement besitzt alle Vorteile der Calciumhydroxiddispersionen, d.h. er bewirkt die Bildung von Sekundärdentin und wirkt durch seinen hohen pH-Wert von mehr als 11 als Schutzsperre gegen Säuren und andere toxische Substanzen, die in einigen Füllmaterialien enthalten sind. Durch das Vorhandensein der Salicylsäureester erhärtet der Zement nach dem Eindringen in die Zahnhöhle schnell zu einer Unterfüllung, welche die Zahnpulpa während des späteren Einbringens der Füllung schützt.

Ein weiterer Dentalzement für eine Unterfüllung, der aus Calciumhydroxid und einem Salicylsäureester eines einwertigen Alkohols besteht, ist in der CH-A-610 199 beschrieben. Aus diesem Zement hergestellte Unterfüllungen besitzen eine relativ geringe Druckfestigkeit.

Es hat sich jedoch gezeigt, dass Dentalzemente auf Basis von Calciumhydroxid und Salicylsäureestern zwar nach ihrer Aufbringung die erläuterten vorteilhaften Eigenschaften zeigen, dass sie jedoch relativ wasserlöslich sind und infolgedessen ausgewaschen werden.

Dieser nachteilige Effekt ist beispielsweise in The Journal of the British Dental Association, 147 (5) 1979, Seite 111, beschrieben worden.

Untersuchungen der Anmelderin haben bestätigt, dass der gemäss US-A-3 047 408 hergestellte Zement bei der Lagerung in Wasser in relativ kurzer Zeit zerfällt.

Es war bekannt, zur Verminderung der Wasserlöslichkeit Paraffinöl oder Leinöl zu Zementen auf Basis von Salicylsäureestern zuzusetzen. Durch diesen Zusatz wird die Wasserfestigkeit zwar verbessert, es stellt sich aber nur ein pH-Wert von weniger als 10 ein, wodurch der vorteilhafte Pulpenschutz verloren geht. Bei allen bisher bekannten Versuchen zur Herstellung eines Calciumhydroxid-enthaltenden Zements schliessen sich hoher pH-Wert und Wasserbeständigkeit gegenseitig aus.

Die Dentalzemente auf Basis von Calciumhydroxid und Salicylsäureestern werden üblicherweise aus zwei Pasten erhalten: einer sog. Salicylesterpaste, deren Hauptbestandteil eine Mischung verschiedener Salicylester mit inerten Füllstoffen ist, und einer sog. Calciumhydroxidpaste, die als Hauptbestandteil Calciumhydroxid in einer flüssigen Pastengrundlage enthält.

Ein weiterer Nachteil der bisher üblichen Calciumhydroxidzemente ist die Entmischung der pastösen Komponente, welche die Ester enthält, vor ihrer Anwendung.

Aus der GB-A-2 041 954 sind Dentalzemente zur Pulpenüberkappung und zum Auskleiden von Kavitäten bekannt, die aus Calciumhydroxid oder Calciumoxid und einem harzartigen Kondensationsprodukt aus einem Salicylsäureester mit Formaldehyd oder Acetaldehyd bestehen, wobei das Kondensationsprodukt in Form eines Gemisches mit Salicylsäureester zur Anwendung kommen kann.

Der Erfindung liegt die Aufgabe zugrunde, einen Dentalzement auf Basis von Calciumhydroxid und Salicylsäureestern von aliphatischen Alkoholen zur Verfügung zu stellen, der einen ausreichend hohen pH-Wert hat und die Bildung von Sekundärdentin fördert und daher seine Funktion als Schutzsperre gegen Säuren und andere toxische Substanzen voll erfüllt, der andererseits jedoch gute Wasserbeständigkeit und erhöhte Druckfestigkeit aufweist und damit dauerhaften Pulpenschutz gewährleistet.

Es wurde gefunden, dass diese Aufgabe dadurch gelöst werden kann, dass man Polyvinylbutyral zu einem Dentalzement auf der Basis von Salicylsäureestern und Calciumhydroxid zusetzt.

Aus der DE-A-1 006 125 war es bekannt, Polymerfilme zum Schutz der Zahnpulpa durch Aufbringen einer organischen Lösung eines Polymeren und Abdunsten des Lösungsmittels herzustellen. Eine Verwendung dieser Polymeren als Zusatz zu alkalischen, selbsthärtenden Unterfüllungszementen ist jedoch nicht vorgesehen. Zu der grossen Zahl der erwähnten Polymeren gehören Polymere der verschiedensten Polymerklassen, wobei Polystyrol und Polyvinylacetat bevorzugt sind.

Ein Hinweis auf Polyvinylbutyral wird nicht gegeben.

Wie aus den später beschriebenen Beispielen und Vergleichsbeispielen hervorgeht, sind Polymere, die in der DE-A-1 006 125 zur Herstellung von Polymerfilmen beschrieben werden, zur Lösung der erfindungsgemässen Aufgabe überhaupt nicht geeignet, da sie nicht die erforderliche Verbesserung der Wasserbeständigkeit von Dentalzementen auf Basis von Salicylsäureestern und Calciumhydroxid verursachen.

Polyvinylbutyral ist dagegen als Bindemittel in Gummidruckfarben und Lacken bekannt. Diese Verwendung liess nicht vermuten, dass der erfindungsgemässe Zusatz von Polyvinylbutyral zu einem Calciumhydroxidzement dessen Eigenschaften verbessert.

Gegenstand der Erfindung ist eine Zahnfüllmasse mit dem Zusatz eines Polymeren, bestehend aus zwei voneinander getrennt vorliegenden Anmischkomponenten, wobei eine Anmischkomponente a) Salicylsäureester aliphatischer Alkohole und die andere Anmischkomponente b) Calciumhydroxid enthält, die dadurch gekennzeichnet ist, dass einer oder beiden Anmischkomponenten Polyvinylbutyral mit einem Acetalisierungsgrad von mindestens 75% zugesetzt ist.

Die durch den erfindungsgemässen Zusatz von Polyvinylbutyral erhaltenen Dentalzemente besitzen geringe Wasserlöslichkeit und haben nach ihrer Härtung erhöhte Druckfestigkeit.

Auch der pH-Wert bleibt stark alkalisch, so dass ein wirksamer Pulpenschutz erreicht werden kann und die Bildung einer Sekundärdentinschicht angeregt wird.

Der erfindungsgemässe Zusatz von Polyvinylbutyral fördert ausserdem die Entmischungsbeständigkeit der Ester-Paste, d.h. der Mischungskomponente für den Dentalzement, welche die Salicylsäureester enthält.

Mit Ausnahme des erfindungsgemässen Zusatzes von Polyvinylbutyral entspricht die Zementzusammensetzung der von bekannten Salicylsäureester-Calciumhydroxid-Dentalzementen, wie sie beispielsweise in der US-A-3 047 408 und der ähnlichen DE-A-1 202 937 beschrieben sind.

Einer der Hauptbestandteile ist Calciumhydroxid, das vorzugsweise in einem stöchiometrischen Überschuss gegenüber dem oder den Salicylsäureestern vorhanden ist.

Ein weiterer wesentlicher Bestandteil ist ein Salicylsäureester eines ein- oder mehrwertigen Alkohols; vorzugsweise eines aliphatischen Alkohols mit 1 bis 8 Kohlenstoffatomen, insbesondere eines solchen zwei- oder dreiwertigen aliphatischen Alkohols, vorzugsweise mit 3–8 Kohlenstoffatomen.

Dabei können Gemische aus zwei oder mehr verschiedenen solcher Ester vorliegen.

Gemäss einer bevorzugten Ausführungsform werden Salicylsäureester von mehrwertigen Alkoholen mit 3 bis 8 Kohlenstoffatomen zusammen mit Salicylsäureestern von einwertigen aliphatischen Alkoholen mit 1 bis 4 Kohlenstoffatomen, insbesondere Methyl- oder Ethylester, eingesetzt.

Eine besonders bevorzugte Ausführungsform enthält ein Gemisch aus dem Salicylsäuremonoester und -diester von 1,3-Butandiol und Salicylsäuremethylester.

Weiterhin kann der Dentalzement übliche inerte Füllstoffe, wie Titandioxid, Zinndioxid, Calciumsulfat, Calciumphosphat, Aluminiumoxid, Kieselsäure oder Zinkoxid enthalten und darüber hinaus können Pigmente, Röntgenkontrastmittel, wie Bariumsulfat oder Calciumwolframat, Weichmacher und sonstige für Füllmaterialien übliche Zusätze vorhanden sein, wie z.B. Zinkstearat.

Das erfindungsgemäss zugesetzte Polyvinylbutyral wird vorteilhaft in einer Menge von 0,2 bis 5 Gew.-%, vorzugsweise 0,5 bis 2 Gew.-% bezogen auf das Gewicht des oder der Ester, zugemischt. Es hat einen Acetalisierungsgrad von mindestens 75% und vorzugsweise mindestens 80% oder darüber. Der Polymerisationsgrad beträgt 20–70, bevorzugt 50–70.

Für die Zwecke der Erfindung eignet sich handelsübliches Polyvinylbutyral, das beispielsweise in der Firmenschrift der Farbwerke Hoechst AG «Mowital B» unter der Bezeichnung Mowital B 20 H, Mowital B 30 H und Mowital B 60 HH beschrieben ist. Solange der gewünschte Acetalisierungsgrad erreicht wird, eignen sich selbstverständlich auch andere Handelsprodukte.

Die Grundmaterialien zur Herstellung eines Dentalzements auf Basis von Salicylsäureestern und Calciumhydroxid werden gemäss einer bevorzugten Ausführungsform als Zweipastensystem hergestellt und vertrieben: Einer Calciumhydroxidpaste und einer Salicylesterpaste, die kurz vor der Anwendung miteinander angemischt werden. Die Aushärtung geschieht durch Eindringen von Feuchtigkeit, die im Mund in ausreichender Menge vorhanden ist, wobei die Körpertemperatur die Aushärtung beschleunigt.

Die Calciumhydroxid-Paste enthält in bekannter Form als Hauptbestandteil Calciumhydroxid. Zusätzlich können Füllstoffe wie Titandioxid, Zinndioxid, Calciumsulfat, -phosphat, Aluminiumoxid, Kieselsäure und Zinkoxid, oberflächenaktive Substanzen wie z.B. Zinkstearat und als flüssige Grundlage ein Weichmacher, wie z.B. Ethyltoluolsulfonamid vorhanden sein.

Die Esterpaste kann aus einem Gemisch der Salicylsäureester, inerten Füllstoffen, wie Titandioxid, Zinndioxid, Calciumphosphat, Zinkoxid, Kieselsäure, Aluminiumoxid etc., und einem Röntgenopaker wie z.B. Calciumwolframat bestehen. Weiterhin kann der Esterpaste eine geringe Menge Calciumhydroxid zugesetzt werden.

Gegebenenfalls können beide Pasten zusätzlich Pigmente enthalten.

Das erfindungsgemäss verwendete Polyvinylbutyral wird vorzugsweise der Esterpaste zugemischt, da es bei Verwendung von Füllstoffen in dieser Paste eine Entmischung von Flüssigkeit und Feststoff weitgehend verhindert. Grundsätzlich kann das Polyvinylbutyral jedoch jeder der beiden Komponenten zugegeben werden oder auch auf beide Komponenten aufgeteilt werden.

Beispiel 1
Herstellung der Esterpaste

In einem Gemisch aus 78 g 1,3-Butandioldisalicylsäureester (Diester), 12,9 g 1,3-Butandiolmonosalicylsäureester (Monoester) und 8,4 g Salicylsäuremethylester werden 0,8 g Polyvinylbutyral, hochacetalisiert (Acetalisierungsgrad ca. 82%) gelöst. 39,3 g dieser Lösung werden mit 60,7 g einer Pulvermischung aus 0,6 g Calciumhydroxid, 15,2 g Calciumwolframat und 45,1 g Titandioxid zu einer Paste vermischt.

Herstellung der Calciumhydroxid-Paste

40,7 g Calciumhydroxid werden mit 8,2 g Zinkoxid, 4,5 g Titandioxid, 0,3 g Zinkstearat, 7 g Kieselsäure und 39,4 g N-Ethyl-(o,p)toluolsulfonamid zu einer Paste vermischt.

Herstellung des Dentalzements

Gleiche Volumina Esterpaste und Calciumhydroxidpaste werden zu einem zahnzementartigen Gemisch verarbeitet, das nach der Erhärtung bei 100% relativer Luftfeuchte und 36 °C die in der Tabelle angegebenen physikalischen Eigenschaften besitzt.

Vergleichsbeispiel 1

Ein Zahnzement wird aus den gleichen Komponenten wie in Beispiel 1 hergestellt, wobei jedoch der Zusatz von Polyvinylbutyral weggelassen wird.

Die physikalischen Eigenschaften des erhaltenen, gehärteten Zements werden in gleicher Weise wie in Beispiel 1 gemessen und sind in der Tabelle aufgeführt.

Vergleichsbeispiel 2

Ein Zahnzement wird aus den gleichen Komponenten wie in Beispiel 1 hergestellt, wobei jedoch dem Salicylsäureestergemisch 1,9% Polyvinylacetat (Vinnapas UW 100) zugesetzt werden.

Die physikalischen Eigenschaften des erhaltenen gehärteten Zements, die in gleicher Weise wie in Beispiel 1 gemessen werden, sind in der nachstehenden Tabelle aufgeführt.

Vergleichsbeispiel 3

Ein Zahnzement wird aus den gleichen Komponenten wie in Beispiel 1 hergestellt, wobei jedoch dem Salicylsäureestergemisch 1,6% Poly-n-butylmethacrylat zugesetzt werden.

Die physikalischen Eigenschaften des erhaltenen gehärteten Zements, die in gleicher Weise wie in Beispiel 1 gemessen wurden, sind in der nachstehenden Tabelle aufgeführt.

Beispiel 2

Herstellung der Esterpaste

In einem Gemisch aus 77,8 g Diester (s. Beispiel 1), 12,85 g Monoester (s. Beispiel 1) und 8,54 g Salicylsäuremethylester werden 0,8 g Polyvinylbutyral (Acetalisierungsgrad ca. 82%) gelöst. 39,3 g dieser Lösung werden mit 60,7 g einer Pulvermischung aus 0,6 g Calciumhydroxid, 15,1 g Calciumwolframat und 45 g Aluminiumoxid zu einer Paste verarbeitet.

Herstellung des Dentalzements

Gleiche Volumina dieser Esterpaste werden mit einer Calciumhydroxidpaste nach Beispiel 1 zu einem zahnzementartigen Gemisch verarbeitet, das nach der Erhärtung vergleichbare Eigenschaften wie der Zement aus Beispiel 1 besitzt.

Beispiel 3

Herstellung der Esterpaste

79 g Diester (s. Beispiel 1), 12,9 g Monoester (s. Beispiel 1) und 8,5 g Salicylsäuremethylester werden vermischt.

39,1 g dieser Lösung werden mit 45,1 g Titandioxid, 15,2 g Calciumwolframat und 0,6 g Calciumhydroxid zu einer Paste verarbeitet.

Herstellung der Calciumhydroxid-Paste

40,7 g Calciumhydroxid werden mit einer Lösung von 0,8 g Polyvinylbutyral (Mowital B 60 HH) in 43,3 g N-Ethyl-(o,p)toluol-sulfonamid und 8,2 g Zinkoxid, 0,25 g Zinkstearat, 2,3 g pyrogener Kieselsäure und 4,5 g Titandioxid zu einer Paste verarbeitet.

Herstellung des Zahnzementes

Gleiche Volumina beider Pasten werden vermischt, nach dem Aushärten bei 100% Luftfeuchtigkeit und 36 °C ergibt sich ein Zement, der als wasserfeste, alkalische Unterfüllung geeignet ist.

Beispiel 4

Herstellung der Esterpaste

In einer Mischung aus 76,7 g Diester (s. Beispiel 1), 12,6 g Monoester (s. Beispiel 1) und 8,3 g Salicylsäuremethylester werden 2,4 g Polyvinylbutyral gelöst.

39,1 g dieser Lösung werden mit einer Pulvermischung aus 45,1 g Titandioxid, 15,2 g Calciumwolframat und 0,6 g Calciumhydroxid zu einer pastösen Masse verarbeitet.

Herstellung des Zahnzements

Durch Vermischen eines Volumenteils dieser Paste mit einem Volumenteil der Calciumhydroxid-Paste nach Beispiel 1, wird ein Zement mit den dort genannten Eigenschaften erhalten.

Beispiel 5

Herstellung der Esterpaste

In einer Mischung aus 78 g Diester (s. Beispiel 1), 12,9 g Monoester (s. Beispiel 1), 13,8 g Trimethylolpropan-tris-salicyl-säureester und 8,4 g Salicylsäure-methylester werden 1,2 g hochacetalisiertes Polyvinylbutyral (Acetalisierungsgrad ca. 82%) gelöst.

22 g dieser Mischung werden mit einem Pulvergemisch aus 0,3 g Calciumhydroxid, 7,6 g Calciumwolframat und 22,6 g Titandioxid zu einer homogenen Paste vermischt.

Herstellung des Dentalzements

Ein Volumenteil dieser Esterpaste wird mit einem Volumenteil der in Beispiel 1 hergestellten Calciumhydroxidpaste zu einem homogenen Zement verarbeitet, der nach seiner Aushärtung günstige Eigenschaften als Unterfüllungszement aufweist.

Beispiel 6

Herstellung der Esterpaste

38,6 g der Lösung von Polyvinylbutyral im Salicylestergemisch nach Beispiel 2 werden mit 14,8 g Calciumwolframat, 0,6 g Calciumhydroxid, 44,1 g Zinndioxid und 2 g pyrogener Kieselsäure zu einer Paste vermischt.

Herstellung des Zahnzements

Durch Vermischen eines Volumenteils dieser Esterpaste mit einem Volumenteil der Calciumhydroxidpaste nach Beispiel 1 wird ein Unterfüllungszement mit den dort genannten Eigenschaften erhalten.

Tabelle

| Beispiel | Zusatz | Druck-festigkeit (1) (kp/cm²) | Zeit bis Druck-festigkeit unmessbar (2) (Wochen) | Löslichkeit 24 h i. $H_2O$ (mg) | Entmischung der Ester-paste |
|---|---|---|---|---|---|
| Beispiel 1 | 0,8 % Polyvinylbutyral (Mowital B60 HH) | 260 | > 24 | 7,5 | keine |
| Vergleichs-beisp. 1 | ohne | 100 | 6 | 19 | deutliche Separierung |
| Vergleichs-beisp. 2 | 1,9 % Polyvinylacetat (Vinnapas UW 100) | 96 | 12 | 40 | deutliche Separierung |
| Vergleichs-beisp. 3 | 1,6 % Poly-n-butylmeth-acrylat | 228 | 12 | 17 | starke Sepa-rierung |

(1): nach 24 Stunden Lagerung bei 36 °C und 100% relativer Feuchtigkeit
(2): unter Wasser bei 36 °C bis zur Auflösung

**Patentansprüche für die Vertragsstaaten: BE, CH, DE, FR, GB, IT, LI, LU, NL**

1. Zahnfüllmasse mit dem Zusatz eines Polymeren, bestehend aus zwei voneinander getrennt vorliegenden Anmischkomponenten, wobei eine Anmischkomponente a) Salicylsäureester aliphatischer Alkohole und die andere Anmischkomponente b) Calciumhydroxid enthält, dadurch gekennzeichnet, dass einer oder beiden Anmischkomponenten Polyvinylbutyral mit einem Acetalisierungsgrad von mindestens 75% zugesetzt ist.

2. Zahnfüllmasse nach Anspruch 1, dadurch gekennzeichnet, dass das zugesetzte Polyvinylbutyral einen Acetalisierungsgrad von mindestens 80% hat.

3. Zahnfüllmasse nach Anspruch 1 oder 2, dadurch gekennzeichnet, dass die Komponente a) Salicylsäureester mehrwertiger aliphatischer Alkohole enthält.

4. Zahnfüllmasse nach Anspruch 1 oder 2, dadurch gekennzeichnet, dass die Anmischkomponente a) Salicylsäureester ein- und mehrwertiger Alkohole enthält.

5. Zahnfüllmasse nach einem der Ansprüche 1 bis 4, dadurch gekennzeichnet, dass 0,2 bis 5 Gew.-% Polyvinylbutyral, bezogen auf das Gewicht des oder der Salicylsäureester in der Zahnfüllmasse, zugesetzt ist.

6. Zahnfüllmasse nach einem der Ansprüche 1 bis 5, dadurch gekennzeichnet, dass die Anmischkomponente a) gegebenenfalls übliche Füllstoffe und Pigmente sowie gegebenenfalls andere übliche Zusätze enthält.

**Patentansprüche für den Vertragsstaat AT**

1. Zahnfüllmasse mit dem Zusatz eines Polymeren, bestehend aus zwei voneinander getrennt vorliegenden Anmischkomponenten, wobei eine Anmischkomponente a) Salicylsäureester aliphatischer Alkohole und die andere Anmischkomponente b) Calciumhydroxid enthält, dadurch gekennzeichnet, dass einer oder beiden Anmischkomponenten Polyvinylbutyral mit einem Acetalisierungsgrad von mindestens 75% zugesetzt ist.

2. Zahnfüllmasse nach Anspruch 1, dadurch gekennzeichnet, dass das zugesetzte Polyvinylbutyral einen Acetalisierungsgrad von mindestens 80% hat.

3. Zahnfüllmasse nach Anspruch 1 oder 2, dadurch gekennzeichnet, dass die Komponente a) Salicylsäureester mehrwertiger aliphatischer Alkohole enthält.

4. Zahnfüllmasse nach Anspruch 1 oder 2, dadurch gekennzeichnet, dass die Anmischkomponente a) Salicylsäureester ein- und mehrwertiger Alkohole enthält.

5. Zahnfüllmasse nach einem der Ansprüche 1 bis 4, dadurch gekennzeichnet, dass 0,2 bis 5 Gew.-% Polyvinylbutyral, bezogen auf das Gewicht des oder der Salicylsäureester in der Zahnfüllmasse, zugesetzt ist.

6. Zahnfüllmasse nach einem der Ansprüche 1 bis 5, dadurch gekennzeichnet, dass die Anmischkomponente a) gegebenenfalls übliche Füllstoffe und Pigmente sowie gegebenenfalls andere übliche Zusätze enthält.

7. Verfahren zur Herstellung einer Zahnfüllmasse mit dem Zusatz eines Polymeren, bestehend aus zwei voneinander getrennt vorliegenden Anmischkomponenten, wobei eine Anmischkomponente (a) Salicylsäureester aliphatischer Alkohole und die andere Anmischkomponente (b) Calciumhydroxid enthält, dadurch gekennzeichnet, dass man einer oder beiden Anmischkomponenten Polyvinylbutyral mit einem Acetalisierungsgrad von mindestens 75% zusetzt.

8. Verfahren nach Anspruch 7, dadurch gekennzeichnet, dass man Polyvinylbutyral mit einem Acetalisierungsgrad von mindestens 80% zusetzt.

9. Verfahren nach Anspruch 7 oder 8, dadurch gekennzeichnet, dass man das Polyvinylbutyral einer Zahnfüllmasse zusetzt, in der die Komponente (a) Salicylsäureester mehrwertiger aliphatischer Alkohole oder Salicylsäureester einwertiger und mehrwertiger Alkohole enthält.

10. Verfahren nach einem der Ansprüche 7 bis 9, dadurch gekennzeichnet, dass man 0,2 bis 5 Gew.-% Polyvinylbutyral, bezogen auf das Gewicht des oder der Salicylsäureester in der Zahnfüllmasse, zusetzt.

**Claims for the contracting states: BE, CH, DE, FR, GB, IT, LI, NL, SE**

1. Tooth filling composition with the addition of a polymer, consisting of two separate mixing components, one mixing component a) containing salicylic acid esters of aliphatic alcohols and the other mixing component b) containing calcium hydroxide, characterized in that polyvinylbutyral with a degree of acetalization of at least 75% has been added to one or both mixing components.

2. Tooth filling composition according to Claim 1, characterized in that the polyvinylbutyral added has a degree of acetalization of at least 80%.

3. Tooth filling composition according to Claim 1 or 2, characterized in that the component a) contains salicylic acid esters of polyhydric aliphatic alcohols.

4. Tooth filling composition according to Claim 1 or 2, characterized in that the mixing component a) contains salicylic acid esters of monohydric and polyhydric alcohols.

5. Tooth filling composition according to one of Claims 1 to 4, characterized in that 0.2 to 5% by weight of polyvinylbutyral, relative to the weight of the salicylic acid ester or salicylic esters in the tooth filling composition, has been added.

6. Tooth filling composition according to one of Claims 1 to 5, characterized in that the mixing component a) optionally contains conventional fillers and pigments and also optionally contains other conventional additives.

**Claims for the contracting state: AT**

1. Tooth filling composition with the addition of a polymer, consisting of two separate mixing components, one mixing component a) containing salicylic acid esters of aliphatic alcohols and the other mixing component b) containing calcium hydroxide, characterized in that polyvinylbutyral with a degree of acetalization of at least 75% has been added to one or both mixing components.

2. Tooth filling composition according to Claim 1, characterized in that the polyvinylbutyral added has a degree of acetalization of at least 80%.

3. Tooth filling composition according to Claim 1 or 2, characterized in that the component a) contains salicylic acid esters of polyhydric aliphatic alcohols.

4. Tooth filling composition according to Claim 1 or 2, characterized in that the mixing component a) contains salicylic acid esters of monohydric and polyhydric alcohols.

5. Tooth filling composition according to one of Claims 1 to 4, characterized in that 0.2 to 5% by weight of polyvinylbutyral, relative to the weight of the salicylic acid ester or salicylic esters in the tooth filling composition, has been added.

6. Tooth filling composition according to one of Claims 1 to 5, characterized in that the mixing component a) optionally contains conventional fillers and pigments and also optionally contains other conventional additives.

7. Process for the preparation of a tooth filling composition with the addition of a polymer, consisting of two separate mixing components, one mixing component a) containing salicylic acid esters of aliphatic alcohols and the other mixing component b) containing calcium hydroxide, characterized in that polyvinylbutyral with a degree of acetalization of at least 75% is added to one or both mixing components.

8. Process according to Claim 7, characterized in that polyvinylbutyral with a degree of acetalization of at least 80% is added.

9. Process according to Claim 7 or 8, characterized in that the polyvinylbutyral is added to a tooth filling composition in which the component a) contains salicylic acid esters of polyhydric aliphatic alcohols or salicylic acid esters of monohydric and polyhydric alcohols.

10. Process according to one of Claims 7 to 9, characterized in that 0.2 to 5% of weight of polyvinylbutyral, relative to the weight of the salicylic acid ester or salicylic acid esters in the tooth filling composition is added.

**Revendications pour les Etats Contractants BE, CH, DE, FR, GB, IT, LI, NL, SE**

1. Composition d'obturation dentaire avec addition d'un polymère, constituée de deux composants de mélange fournis séparément, l'un des composants de mélange a) contenant un salicylate d'alcool aliphatique et l'autre composant de mélange b) contenant de l'hydroxyde de calcium, caractérisée en ce qu'on ajoute, à l'un ou aux deux des composants de mélange, du polyvinylbutyral avec un degré d'acétalisation d'au moins 75%.

2. Composition d'obturation dentaire selon la revendication 1, caractérisée en ce que le polyvinylbutyral ajouté présente un degré d'acétalisation d'au moins 80%.

3. Composition d'obturation dentaire selon la revendication 1 ou 2, caractérisée en ce que le composant a) contient un salicylate d'alcool aliphatique polyvalent.

4. Composition d'obturation dentaire selon la revendication 1 ou 2, caractérisée en ce que le composant a) contient un salicylate d'alcool aliphatique monovalent et polyvalent.

5. Composition d'obturation dentaire selon l'une des revendications 1 à 4, caractérisée en ce que l'on ajoute 0,2 à 5% en poids de polyvinylbutyral, par rapport au poids du ou des salicylates présents dans la composition d'obturation dentaire.

6. Composition d'obturation dentaire selon l'une des revendications 1 à 5, caractérisée en ce que le composant de mélange a) contient éventuellement des charges et pigments habituels ainsi qu'éventuellement d'autres additifs courants.

**Revendications pour l'Etat Contractant AT**

1. Composition d'obturation dentaire avec addition d'un polymère, constituée de deux composants de mélange fournis séparément, l'un des composants de mélange a) contenant un salicylate d'alcool aliphatique et l'autre composant de mélange b) contenant de l'hydroxyde de calcium, caractérisée en ce qu'on ajoute, à l'un ou aux deux des composants de mélange, du polyvinylbutyral avec un degré d'acétalisation d'au moins 75%.

2. Composition d'obturation dentaire selon la revendication 1, caractérisée en ce que le polyvinylbutyral ajouté présente un degré d'acétalisation d'au moins 80%.

3. Composition d'obturation dentaire selon la revendication 1 ou 2, caractérisée en ce que le composant a) contient un salicylate d'alcool aliphatique polyvalent.

4. Composition d'obturation dentaire selon la revendication 1 ou 2, caractérisée en ce que le composant a) contient un salicylate d'alcool aliphatique monovalent et polyvalent.

5. Composition d'obturation dentaire selon l'une des revendications 1 à 4, caractérisée en ce que l'on ajoute 0,2 à 5% en poids de polyvinylbutyral, par rapport au poids du ou des salicylates présents dans la composition d'obturation dentaire.

6. Composition d'obturation dentaire selon l'une des revendications 1 à 5, caractérisée en ce que le composant de mélange a) contient éventuellement des charges et pigments habituels ainsi qu'éventuellement d'autres additifs courants.

7. Procédé pour la préparation d'une composition d'obturation dentaire avec addition d'un polymère, constituée de deux composants de mélange fournis séparément, l'un des composants de mélange a) contenant un salicylate d'alcool aliphatique et l'autre composant de mélange b) contenant de l'hydroxyde de calcium, caractérisée en ce qu'on ajoute, à l'un des composants de mélange ou aux deux composants de mélange, du polyvinylbutyral avec un degré d'acétalisation d'au moins 75%.

8. Procédé selon la revendication 7, caractérisée en ce que l'on ajoute du polyvinylbutyral présentant un degré d'acétalisation d'au moins 80%.

9. Procédé selon la revendication 7 ou 8, caractérisée en ce que l'on ajoute le polyvinylbutyral à une composition d'obturation dentaire dans laquelle le composant a) contient un salicylate d'alcool aliphatique monovalent et polyvalent.

10. Procédé selon l'une des revendications 7 à 9, caractérisée en ce que l'on ajoute 0,2 à 5% en poids de polyvinylbutyral, par rapport au poids du ou des salicylates présents dans la composition d'obturation dentaire.